# EUROPEAN PATENT APPLICATION

(11) **EP 4 710 896 A1**
(43) Date of publication of application: **18.03.2026**
(21) Application number: 23936509.1
(22) Date of filing: 08.05.2023
(51) Int. Cl.: A61F 2/30, A61F 2/28, A61B 17/80, A61B 17/82

(54) **IMPLANT FOR THE STERNOCLAVICULAR JOINT**

(71) Applicant: INSTITUTO TECNOLÓGICO DE CANARIAS, S.A., 35003 Las Palmas de Gran Canaria (ES)
(72) Inventor: MONOPOLI FORLEO, Donato, 35003 LAS PALMAS DE GRAN CANARIA-LAS PALMAS (ES); CANO GARCIA, José Ramón, 35003 LAS PALMAS DE GRAN CANARIA-LAS PALMAS (ES)
(74) Representative: Urizar Anasagasti, Jesus Maria
(86) International application number: PCT/ES2023/070289
(87) International publication number: WO 2024/231581

(57) **Abstract**

The invention relates to an implant for the sternoclavicular joint, said implant being formed by a clavicular component (2), fixed on the sectioned clavicle(s), after surgically removing the clavicular joint; a manubrium component (1) that is fixed or with which the sternal manubrium is reconstructed; joint heads (3) that form the joints of the respective clavicular components (2) on the manubrium component (1); and ligaments (5) that thread the front parts on both sides, left and right, forming a sternoclavicular implant. Optionally, the manubrium component (1) extends towards the lower portion until it is located over a portion of the sternum and it has respective side prolongations coinciding with the ribs C2.

## Description

### Technical field

The field of the invention is that of surgical instruments, devices or methods, for example, cortical plates, or bone plates; instruments for holding or positioning cortical plates, or for compressing bones attached to cortical plates, fastening elements for the same, or fastening elements which are internal fixation devices. More specifically, the object of the invention is an implant for the sternoclavicular joint.

The sternoclavicular joint is a joint formed by the clavicle and the upper and side portion of the manubrium (clavicular notch). An articular disc, which moulds to the joint surfaces, is located between the same. It is a synovial joint with reduced mobility, of the reciprocal or saddle joint type, similar to the trapeziometacarpal joint. One of its peculiarities is that its operation is subject to traction rather than compression, unlike the other synovial joints.

### State of the art

Dislocation of the sternoclavicular joint may compress vital structures behind the joint and may require surgical treatment in patients who have joint instability. This joint may also be affected by bone or soft tissue tumours that require en bloc resection with safety margins that often require removal of the joint portion along with part of the manubrium and clavicle. In some cases, it may be necessary to remove larger areas of the thoracic wall (sternum and several ribs) and then proceed with bone and muscle reconstruction to ensure proper protection of the organs of the thorax and preserve normal respiratory dynamics in patients.

A number of successful operations have been performed in which the bone elements of the removed thorax have been replaced with implants custom-made for each patient. In general, these implants can repair all or part of the sternum and/or ribs; however, there is no knowledge of any implant designed to replace the sternoclavicular joint, and only a few cases of reconstruction of said sternoclavicular joint by means of grafts have been described in the medical literature, as described, for example, in a biomechanical study by Spencer and Kuhn showing that reconstruction of this joint with a figure-of-eight graft with 2 perforations in the clavicle and 2 perforations in the sternum was superior to intramedullary ligament reconstruction and subclavius tendon reconstruction when comparing graft integrity, load to failure, and medial clavicle translation. This study popularised the concept of a figure-of-eight reconstruction to provide anterior and posterior stabilisation of the joint. More recently, Bruchmann, G., Rossi, L.A. et al. consider reconstruction of the sternoclavicular joint with posterior tibial allograft as a valid option for the treatment of chronic instability.

However, several specific implants have been developed for the reconstruction of the sternum and ribs. For example, document US2014257291 describes an apparatus and technique for internally securing a plurality of bone segments. The device incorporates a plate-like structure stabilising the fracture, said plate being contoured to lie passively against the front face of the human sternum when placed directly on its irregular flat surface, and includes a plurality of methods for fastening said plate on the sternum surface when attached around the under surface of said sternum and tensioned. Document US2020085476 also describes an implant for a human sternum and ribs that can be couplable to a sternum. The rib link can include a distal portion that can be couplable to a rib, a proximal portion that can be couplable to the sternal implant, and a strut that can connect the distal portion to the proximal portion. Document US2021298808 describes an implant for sternum replacement including an upper face, a posterior face, extensions for fitting with the ribs and extensions for fitting with the manubrium, wherein that implant is manufactured in a single piece with high performance engineering polymers, with an anatomical adjustment tailored to each patient. Document CN106109057 describes a metal skeleton device for a sternum, which is adapted to the conditions of thoracic wall separation and clavicle separation caused by a tumour removal operation; a connecting member of the clavicle is fixed to the manubrium of the artificial sternum by means of a movable connecting pin which allows some mobility of said connecting member.

It would therefore be desirable to have an implant that replaces the sternoclavicular joint and could occasionally be combined with other elements that also allows reconstruction of the sternum (all or part) and also the resected ribs, that is also easy to assemble and manufacture, and that is simple to fix on the patient in order to reduce surgical times.

### Explanation of the invention

In order to achieve the proposed objectives, which are mentioned in the previous paragraph, the invention proposes an implant for the sternoclavicular joint, having the features of claim 1.

For the reconstruction of the previously resected sternoclavicular joint, the following are used in this implant: a clavicular component that replaces the medial end of the previously resected clavicle, anchoring it to the remaining clavicular fragment using screws, cerclage, staples, or locked pins; a manubrium component that reconstructs the resected portion of the joint and is anchored to the remaining portion of the manubrium, if present, or directly to another bone present (sternum or ribs) with screws or cerclage; a perforated joint head that acts as a spacer, keeping the two joint surfaces (that of the clavicular component and that of the manubrium) in contact with said head at a distance, adapting to the shape of each other to allow movement on both sides while maintaining contact at all times; a ligament that threads the front parts (clavicular component, head and manubrium) and is knotted keeping the joint constrained. Only one joint has been described, where both left and right joints can be replaced at the same time, and different types of implants and anchorages can be used depending on the resection required by the patient.

In patients in whom in general the sternum and ribs had to be partially cut for the removal of a tumour, this implant also includes a downward prolongation of the manubrium component, which simulates a sternum, and side prolongations have also been coupled to replace the sectioned ribs; all this to provide rigidity and flexibility to this area like a normal thorax.

These prostheses are a good alternative for the correction of small and large anterior thoracic wall defects, complex pathologies, giant thoracic hernias and wall pathologies with joint involvement due to their perfect adaptability, easy fitting, reduced surgical time, as well as providing correct stability without limiting respiratory movements.

### Description of the drawings

As a complement to the present description, and for the purpose of helping to make the features of the invention more readily understandable, said specification is accompanied by a set of drawings which, by way of illustration and not limitation, represent the following:

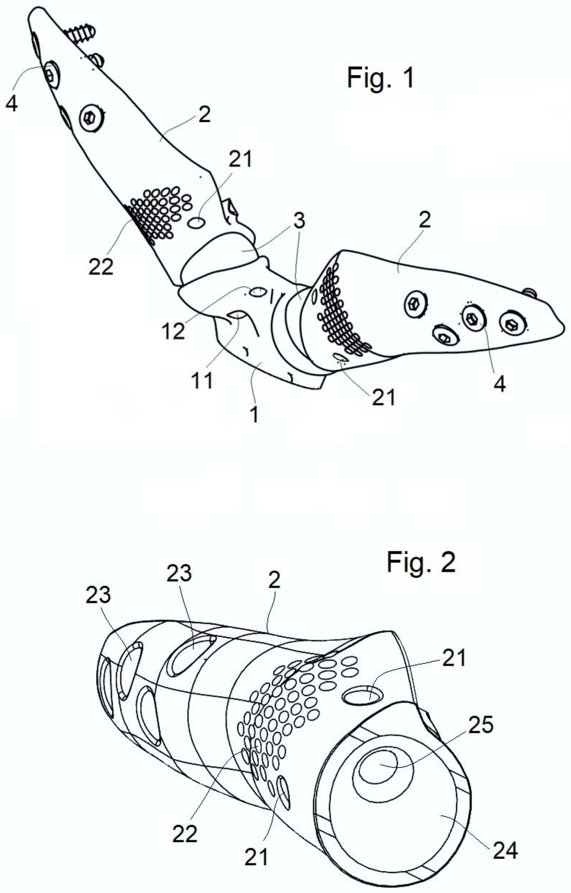

- Figure 1 shows an implant for a sternoclavicular joint.
- Figure 2 shows a front view of the right clavicular part (2).
- Figure 3 shows a view of a complete implant including, in addition to the clavicular joint, the sternum and the two ribs C2.
- Figure 4 represents a front elevation view of the implant in Figure 3.

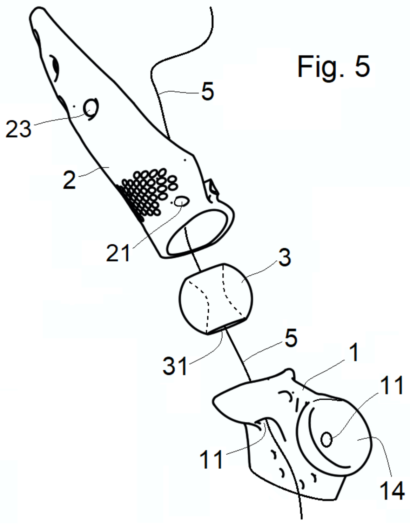

- Figure 5 shows the components of the right portion of an implant for a sternoclavicular joint with its components deployed in the assembled position and to be threaded by a ligament (5).

### Embodiment of the invention

As can be seen in the figures referenced, the implant for the sternoclavicular joint, which has been surgically sectioned, essentially comprises: One clavicular component (2); in the case of both clavicles being sectioned, the clavicular components are symmetrical so that they can be fixed to the respective left and right clavicles. A manubrium component (1), which is fixed to or reconstructs the sternal manubrium. A joint head (3) which, interposed between each clavicular component (2) and the corresponding side of the manubrium component (1), allows its relative mobility in a manner equivalent to a sternoclavicular joint. And, a ligament (5) that threads the components of the same side together. Depending on whether it was necessary to cut one or both clavicles, one or two clavicular components (2) and consequently one or two joint heads (3) will be placed on the side(s) that have been sectioned, those on the same side attached by a ligament (5).

Each of the clavicular components (2), either left or right, comprises:
- On the front face and at the side end, a fixing element for fixing to the bone of the remaining clavicle. This element can be a plate and screws, such as the solution shown in the figures in which holes (23) are arranged through which it is fixed by means of screws (4), after the clavicle bone on which it is placed has been suitably drilled. Other possible alternatives for said element with a plate-type connection and cerclage, pins or clamps.
- On the posterior face of the side portion, there is a concave channel-like surface, which houses the clavicle and ends with a rough base or a porous volume (22) in direct contact with the cut surface of the clavicle, which facilitates integration between the two components.
- At the medial end, there is an essentially hemispherical cavity (24), which accommodates a portion of a joint head (3) through which this portion is connected to the portion attached to the manubrium of the sternum. At the bottom of this cavity is where the through hole (25) for the ligament (5) is located.
- In the inner volume of the component, from the medial portion to the base, there is a tunnel starting with a perforation (25) in the medial hemispherical face (24) that passes through the porous structure (22) and the base.

For its part, the manubrium component (1), which is either fixed or reconstructs the sternal manubrium, has one or two side cavities (14) at the upper portion which house a portion of the respective joint heads (3), which have a hole (11) at the bottom thereof that leads to the front of said component (1) through which the ligament (5) that joins all the parts of the implant exits.

Each of the perforated joint heads (3), which are located in cavities (24) in the bases of the clavicular implants (2) and in the cavities (14) in the side protrusions of the manubrium component (1), are intended to form the joints of the respective clavicular components (2) on the manubrium component (1), which, as already pointed out, can affect one side or the other, or both. These joint heads (3) have an axial perforation or hole (31) through which the ligament (5) also passes so that this part always maintains its position in the joint created. Said perforation may have a cylindrical configuration or be in the form of two cone-shaped trunks attached at their smaller bases; in both cases, said perforation (31) defines respective bases on the surface of the joint head that improves joint movement.

The ligaments (5) thread all the components of the implant through one of the central proximal holes (11) in the manubrium component (1), which then pass through the central hole (31) in the corresponding joint head (3) and finally exit through the hole (25) in the base of the clavicular component (2) until they pass through the bone and exit proximally to the outside.

In a preferred embodiment, it is envisaged that close to the edges of the cavities (24) in the clavicular components (2) and in the cavities (14) of the manubrium component (1), which accommodate each of the joint heads (3), both parts have at least respective pairs of holes (21, 12) on their front face, through which the constrained joint head (3) is fixed by means of a suture thread, which keeps the joint closed and stable while the surgical operation is being performed. In some cases, this connection is removed and in other cases, at the end of the operation, it is reconstructed by reattaching the aforementioned holes by means of respective crossed ligaments that secure both joints.

In a preferred embodiment, it is also envisaged that the hole (25) in the base of each of the clavicular components (2) has a configuration that allows a drill bit to be passed through it to drill a through hole in the clavicle, to create the channel that will subsequently be used to lock the joint with the ligament (5).

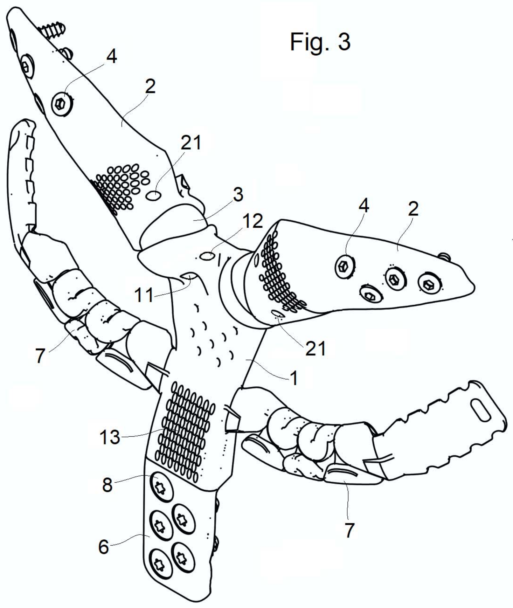

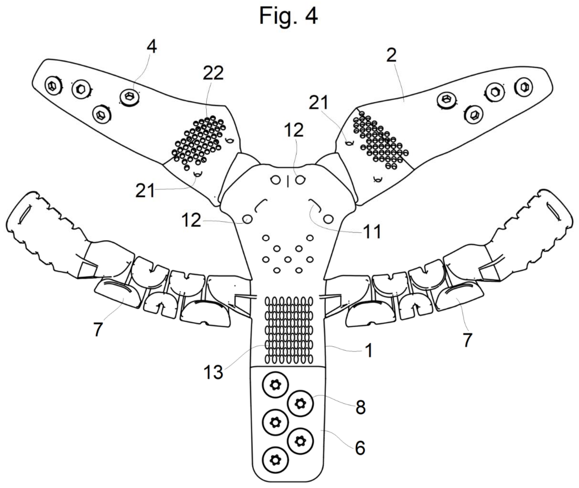

Figures 3-4 show an implant in which the manubrium component (1) has a downward termination until it reaches the non-surgically cut portion of the sternum. Said prolongation has a rough structure or a porous volume (13) resting on the cut made in said bone. In other embodiments, the lower prolongation (6) reaches approximately the entire length of the sternum and is provided with holes through which it is fixed by screws (8) to the sternum.

In these embodiments, in which the manubrium component (1) is elongated downwards, it is also envisaged that it can have respective side prolongations (7) coinciding with the rib walls corresponding to the ribs C2, to which it is attached by means of cerclage.

Although the surgical method is not the subject matter of this application, it is briefly explained below to make it easier to understand the subject matter of the invention. This example will be developed based on the implant in Figures 3-4, which is applied in cases where there is a tumour, the removal of which requires a cut in the sternal body, the first ribs and second ribs and another cut in both clavicles, in order to free the affected area.

The manubrium is first removed by making a cut in the sternal body just above the insertion of the cartilages of the third ribs and perpendicular to the outer surface of the sternum. The first and second ribs are then cut and a guided cut of both clavicles is performed.

The next step is to fix the clavicular implants to the corresponding clavicles with screws. The right clavicular implant and the left clavicular implant are planned to rest with the porous structure (22) directly on the cut surface of the clavicle. To do this, the right clavicle is drilled with a through hole with the drill bit provided to make the channel that will later be used to lock the joint with the ligament. The same operation is repeated with the left clavicle.

For these operations of cutting and drilling the clavicles, guides provided with the implant can be used to mark the exact positions in which both mechanical operations, cutting and drilling, are to be performed.

The component (1) that reconstructs the manubrium is then fixed to the sternum with the screws and to the rib walls corresponding to C2 with cerclage (use of a braided cable system for bone cerclage is recommended). Beforehand, the drilling guide also provided with the implant must be used, positioning it in the hemispherical housings of the implant holes intended for the screws. A 4.4 mm drill bit is threaded into the perforation guide and pre-drilling is carried out with the tip of the mandrel reaching the end of its travel on the perforation guide. The drill bit and the guide are removed and the screw is placed.

One of the ligaments is then threaded consecutively through one of the two central proximal holes in the manubrium component, then through the central hole of the joint component and finally through the hole in the joint of the corresponding clavicle until it passes through the bone and exits proximally to the outside. The joint heads are placed in their spherical housing between the joint portion of the manubrium component-sternum and the clavicular components and the constrained joint portion is maintained using heavy gauge suture thread to help keep the joint closed and stable. This sequence is repeated on the other joint.

Once both joints are locked, one of them must be fixed with the ligament. A figure-of-eight knot is tied at the distal end of the ligament and a few stitches are tied, securing the knot. A suture is placed at the other end of the ligament, the proximal end. The ligament is placed in slight traction and a figure-of-eight knot is tied in the portion close to its exit from the open channel in the bone. The same operation is performed at the other end and finally the proximal portions of the two ligaments are tied together, securing the knots with stitches. A non-absorbable suture is used to suture the knots, avoiding unwanted loosening in the tension of the ligaments.

Lastly, both joints are released by cutting the lock previously made with the suture thread. And, at the surgeon's discretion, other ligaments (bank bone or artificial) are used to better secure both joints according to the sequence described in pink below.

Once the nature of the invention has been described, as well as a preferred exemplary embodiment, it is clear that the invention can be industrially applicable in the indicated sector.

It is also stated for appropriate purposes that the materials, shape, size and arrangement of the elements described may be modified, as long as this does not imply an alteration of the essential features of the invention that are claimed below:

## Claims

1. An implant for the sternoclavicular joint, which has been surgically sectioned, comprising:
- a clavicular component (2), which is fixed to the corresponding sectioned clavicle (left or right), comprising:
∘ on the front face and at the side end, a fixing element for fixing to the bone of the remaining clavicle,
∘ on the posterior face of the side portion, a concave channel-like surface, which houses the clavicle and ends with a rough base or a porous volume (22) in direct contact with the cut surface of the clavicle,
∘ at the medial end, an essentially hemispherical cavity (24), which accommodates a portion of a joint head (3) through which this portion is connected to the portion attached to the manubrium of the sternum, said cavity being provided with a through hole (25) for the ligament (5),
∘ in the inner volume of the component (2), from the medial portion to the base, a tunnel starting with a perforation (25) in the medial hemispherical face (24) and passing through the porous structure (22) and the base;
- a manubrium component (1) that is fixed or reconstructs the sternal manubrium, the upper portion of which has one or two side cavities (14) which house a portion of the respective joint heads (3), which have a hole (11) at the bottom that leads to the front of said component (1) through which the ligament (5) that joins all the parts of the implant exits;
- a perforated joint head (3), located in a cavity (24) in the bases of the clavicular component (2) and in the upper protrusions of the manubrium component (1), which form the joint between both elements;
- a ligament (5), which is threaded through a hole (11) in the manubrium component (1), passes through the central hole (31) in the corresponding joint head (3) and finally exits through the hole (25) in the base of the clavicular component (2) until it passes through the bone and exits proximally to the outside.

2. The implant according to claim 1, wherein, close to the edges of the cavities in the clavicular component (2) and in the manubrium component (1), which accommodate a joint head (3), both parts have at least respective pairs of holes (21, 12) on their front face, through which the constrained joint head (3) is fixed by means of a suture thread to keep the joint closed and stable while the fixation operation with the ligament (5) is being performed.

3. The implant according to claim 2, wherein the aforementioned pairs of holes (21, 12) are linked at the end of the operation to secure both joints by means of respective ligaments.

4. The implant according to any of the preceding claims, wherein the perforation in the joint head (3), which is cylindrical or in the form of two cone-shaped trunks attached at their smaller bases, defines respective bases on the surface of the joint head.

5. The implant according to any of the preceding claims, wherein the hole (25) in the base of the clavicular component (2) has a configuration that allows a drill bit to be passed through it to drill a through hole in the clavicle, to create the channel that will subsequently be used to lock the joint with the ligament (5).

6. The implant according to any of the preceding claims, wherein the manubrium component (1) has a downward termination until it reaches the non-surgically cut portion of the sternum where it has a rough structure or a porous volume (13) resting on the cut made in said bone.

7. The implant according to any of the preceding claims, wherein the manubrium component (1) has a lower prolongation (6) provided with at least one hole through which it is fixed by screws (8) to the sternum.

8. The implant according to any of the preceding claims, wherein the manubrium component (1) has respective side prolongations (7) coinciding with the rib walls corresponding to the ribs C2, to which it is attached by means of cerclage or a screw.
